# EUROPÄISCHE PATENTSCHRIFT

(11) **EP 0 602 411 B1**
(45) Veröffentlichungstag und Bekanntmachung des Hinweises auf die Patenterteilung: **11.06.1997**
(21) Anmeldenummer: 93118795.9
(22) Anmeldetag: 23.11.1993
(51) Int. Cl.: A61B 17/06

(54) **Nahtmaterialpackung**
Suture package
Emballage de suture

(30) Priorität: 04.12.1992 DE 4240832
(43) Veröffentlichungstag der Anmeldung: 22.06.1994
(73) Patentinhaber: B. BRAUN MELSUNGEN AG, D-34212 Melsungen (DE)
(72) Erfinder: Brandau, Rolf, Dipl.-Ing., D-34576 Homberg (DE); Sulzberger, Robert, CH-8200 Schaffhausen (CH)
(74) Vertreter: Selting, Günther, Dipl.-Ing.

(56) Entgegenhaltungen:
- EP-A- 0 521 170
- DE-A- 2 803 409
- US-A- 4 284 194
- US-A- 4 533 041

## Beschreibung

Die Erfindung betrifft eine Nahtmaterialpackung für chirurgisches Nahtmaterial, wobei das Nahtmaterial in einer Faltkarte derart enthalten ist, daß es auf einfache Weise kontrolliert herausgezogen werden kann.

Eine Nahtmaterialpackung, von der der Oberbegriff des Patentanspruchs 1 ausgeht, ist bekannt aus US 4 284 194. Diese Nahtmaterialpackung besteht aus einer Faltkarte, in der ein Fadenbündel aus parallelen Einzelfäden mäanderförmig enthalten ist. Die Enden des Fadenbündels ragen aus einem offenen Ende der Faltkarte heraus. Beim Abziehen eines Einzelfadens kann es vorkommen, daß das gesamte Fadenbündel mitgenommen wird.

Aus US 4 089 409 ist eine Nahtmaterialpackung bekannt, bei der ein Fadenbündel in einer Faltkarte enthalten ist. Durch Übereinanderfalten mehrerer Platten kommen Randaussparungen dieser Platten in eine sich teilweise überdeckende Lage. Durch diese Randaussparungen verläuft das Fadenbündel hindurch. Hierbei soll zwar erreicht werden, daß die Einzelfäden aus dem Fadenbündel abgezogen werden, ohne daß die in der Packung verbleibenden Fäden sich miteinander verwirren, jedoch stellt die Packung nicht sicher, daß Fäden aus der jeweils zum Ende hin völlig offenen Ausnehmung herausgleiten oder daß die Mitnahme anderer Fäden zuverlässig verhindert wird.

Aus US-A-4 533 041 ist eine Nahtmaterialpackung in Form einer Langpackung bekannt, in der das Nahtmaterial in einer einzigen Schleife verlegt wird. In der Nähe der Schleifenenden ist eine abreißbare Lasche mit einer Durchführungsöffnung, von der sich ein Schlitz zum benachbarten Plattenrand erstreckt, vorgesehen. In diese Durchführungsöffnung wird das eine Ende eines Fadenbündels durch den Schlitz hindurch eingeführt. Die Durchführungsöffnung ist so eng bemessen, daß beim Herausziehen eines Einzelfadens aus dem Fadenbündel die anderen Fäden zurückgehalten werden.

In der älteren (nicht vorveröffentlichten) EP 0 521 170 A1 ist eine Nahtmaterialpackung beschrieben, bei der sich im Faltungsbereich zwischen zwei Platten eine Durchtrittsöffnung befindet, durch die die Fadenenden eines mäanderförmig verlegten Fadenbündels hindurchführen. Diese Durchtrittsöffnung, in die ein seitlicher Schlitz mündet, ist einerseits so groß, daß sie ein sicheres Zurückhalten der Fäden nicht gewährleistet, und sie erstreckt sich andererseits innerhalb von zwei Platten.

Der Erfindung liegt die Aufgabe zugrunde, eine Kombination aus Nahtmaterialpackung und Nahtmaterial zu schaffen, die das Wickeln und Fixieren des Nahtmaterials erleichtert und für Fadenbündel geeignet ist und eine Einzelentnahme ermöglicht, ohne daß weitere Fäden unbeabsichtigt mitgeschleppt werden.

Die Lösung dieser Aufgabe erfolgt erfindungsgemäß mit den im Patentanspruch 1 angegebenen Merkmalen.

Bei der erfindungsgemäßen Kombination ist die Faltkarte so ausgebildet, daß das Fadenbündel durch eine Durchführungsöffnung bzw. ein Loch geführt wird. Dieses Loch ist auf den Durchmesser des Fadenbündels abgestimmt. Das Fadenbündel wird durch einen Schlitz vom Rand her in die Durchführungsöffnung eingeführt. Bis auf diesen Schlitz ist die Einführungsöffnung vollständig vom Material der Faltkarte umgeben. Das Fadenbündel ist im Innern der Faltkarte mäanderförmig verlegt. Bei Entnahme eines Einzelfadens wird die mäanderförmige Ablage der Fäden teilweise gestört, wobei benachbarte Fäden in Richtung auf die Durchführungsöffnung mitgenommen werden, jedoch gelangen keine Fäden, an denen nicht von außen gezogen wird, durch die Durchführungsöffnung nach außen. Die Fäden können also einzeln entnommen werden, ohne andere Fäden mitzureißen. Die Nahtmaterialpackung kann somit mehrere Fäden enthalten, die nacheinander benutzt werden. Die mäanderförmige Verlegung des Fadenbündels im Innern der Faltkarte erleichtert das kontrollierte Herausziehen einzelner Fäden.

Das Fadenbündel ist in einer an entgegengesetzten Enden offenen Tasche festgelegt, die von einer Wickelplatte und einer Überfaltplatte gebildet und auf eine Basisplatte umgefaltet ist, wobei die Durchführungsöffnung in der Basisplatte und/oder einer darüber faltbaren Deckplatte vorgesehen ist. Die Durchführungsöffnung sollte zu derjenigen Seite, nach der die Fadenenden aus ihr austreten, offen und nicht von anderen Teilen der Faltkarte bedeckt sein. Zweckmäßigerweise ist eine Fadenhalteplatte vorgesehen, die die Fadenenden auf der Rückseite der Durchführungsöffnung aufnimmt, um diese Fadenenden sowohl beim Wickelvorgang als auch beim Aufreißvorgang in definierter Lage zu halten.

Im folgenden wird unter Bezugnahme auf die Figuren ein Ausführungsbeispiel der Erfindung näher erläutert.

Es zeigen:
- Fig. 1: die Faltkarte im auseinandergefalteten Zustand,
- Fig. 2: eine Einzelheit aus Fig. 1 mit Angabe der Umfaltrichtungen der Fadenhalteplatte und der Faltplatte,
- Fig. 3: eine Ansicht der Faltplatte nach dem Wickelvorgang,
- Fig. 4: das restliche Umfalten der Faltplatte,
- Fig. 5: die fertig gefaltete Faltplatte,
- Fig. 6: das Einbringen der Faltplatte in die Hülle und
- Fig. 7: das Aufreißen der Hülle zum Entnehmen des Fadens.

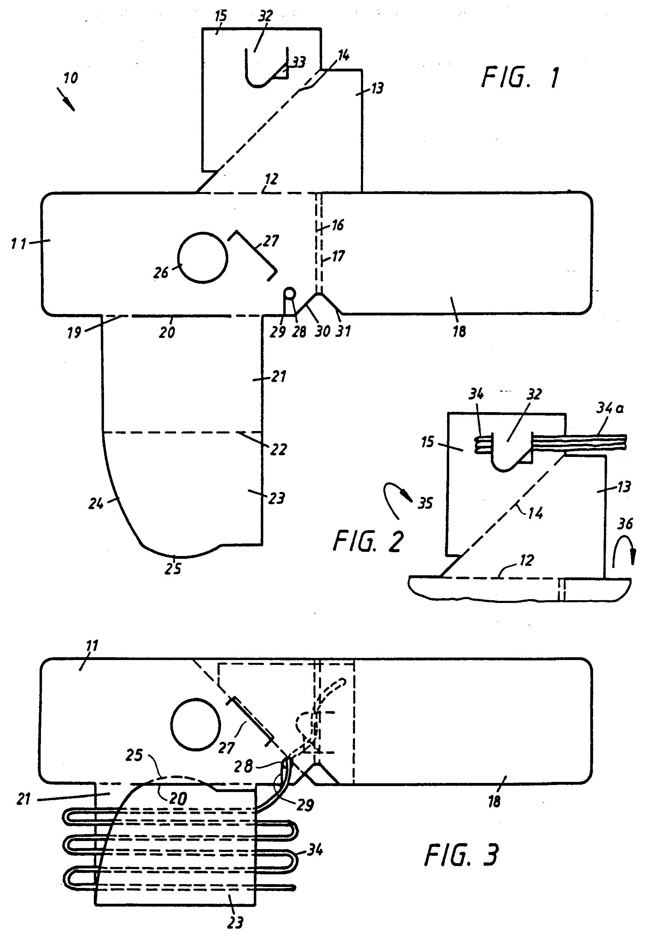

Die in Fig. 1 dargestellte Faltkarte 10 besteht aus einem einstückigen Zuschnitt aus festem Papier oder Karton. Sie weist eine rechteckige, länglich gestaltete Basisplatte 11 auf, deren eine Längskante 12 in Form einer Faltlinie in eine Faltplatte 13 übergeht. Die Faltplatte 13 erstreckt sich über die eine Hälfte der Basisplatte 11 und sie überragt die Basisplatte 11 in Längsrichtung. Die Faltplatte 13 wird durch eine schräg unter 45° zur Längsrichtung der Basisplatte 11 verlaufende Faltlinie 14 begrenzt, deren Verlängerung auf die Längskante 12 der Basisplatte stößt, und an die sich die Fadenhalteplatte 15 anschließt. Die Fadenhalteplatte 15 ist deckungsgleich mit der Faltplatte 13, so daß sie unter die Faltplatte gefaltet werden kann. Beide Platten 13 und 15 sind im wesentlichen dreieckförmig.

An das der Faltplatte 13 benachbarte Ende der Basisplatte 11 schließt sich über zwei parallele Faltlinien 16,17 eine Deckplatte 18 an, die etwa die gleiche Größe hat wie die Basisplatte und über diese gefaltet werden kann.

An der der Längskante 12 abgewandten Längskante der Basisplatten 11 befindet sich eine Faltlinie 19 mit einem Einsteckschlitz 20. An die Faltlinie 19 schließt sich die Wickelplatte 21 an, die an ihrer gegenüberliegenden Kante durch eine Faltlinie 22 begrenzt ist, an welche sich die Überfaltplatte 23 anschließt. Die Wickelplatte 21 und die Überfaltplatte 23 sind kürzer als die Basisplatte 11, die diese beiden Platten 21 und 23 an beiden Enden überragt. Die Überfaltplatte 23 ist im wesentlichen deckungsgleich mit der Wickelplatte 21, jedoch weist sie an einer Kante eine Abrundung 24 und an ihrer äußeren Längskante eine Lasche 25 auf, die in den Einsteckschlitz 20 eingesteckt werden kann.

Die Basisplatte 11 ist mit einem Zentrierloch 26 versehen, in das ein Zentrierdorn einer Wickelmaschine eingesteckt werden kann, um die Faltkarte 10 in der Wickelmaschine zu zentrieren. Ferner ist an der Basisplatte 10 eine U-förmig ausgestanzte Verriegelungslasche 27 vorgesehen, die schräg unter 45° zur Längsrichtung der Basisplatte verläuft und unter die die von der Faltlinie 14 gebildete Faltkante gesteckt werden kann.

In der Nähe der einen Ecke der Basisplatte 11 ist eine Durchführungsöffnung 28 vorgesehen, die vollständig in der Basisplatte enthalten ist und über einen Schlitz 29 in die Längskante hinein ausläuft. Die Durchführungsöffnung 28 befindet sich an der der Faltplatte 13 gegenüberliegenden Ecke. In dieser Ecke der Basisplatte 11 ist eine Abschrägung 30 vorgesehen, und an der angrenzenden Ecke der Deckplatte 18 ist eine Abschrägung 31 vorgesehen. Diese Abschrägungen ermöglichen auch bei nicht genauer Plazierung der gefalteten Faltkarte in der Hülle ein sicheres Aufreißen der Hülle.

An der Fadenhalteplatte 15 ist eine Fixierlasche 32 zum Einklemmen des Fadenendes ausgestanzt. Diese Fixierlasche 32 ist der Basisplatte 11 zugewandt. An einer Kante ist sie durch eine Öffnung 33 begrenzt, die während des Aufreißens der Hülle ein leichtes Herausgleiten des Fadenendes aus der Fixierlasche ermöglicht.

In die Faltkarte 10 wird in dem in Fig. 1 dargestellten Zustand das Fadenende eines aus Fäden 34 bestehenden Fadenbündels 34a gemäß Fig. 2 unter der Fixierlasche 32 fixiert, so daß dieses Fadenende festgeklemmt wird. Die Fadenhaltekarte 15 wird in Richtung des Pfeils 35 um die Faltlinie 14 herum unter die Faltplatte 13 gefaltet, so daß die Fixierlasche 32 sich mit dem Fadenbündel 34a auf der Rückseite befindet. Dann wird die Faltplatte 13 um die Längskante 12 herum in Richtung des Pfeiles 36 unter die Basisplatte 11 gefaltet, und die von der Faltlinie 14 gebildete Kante wird unter die Verriegelungslasche 27 der Basisplatte 11 gesteckt. Die Fixierlasche 32 befindet sich nun mit den Fadenenden auf der Rückseite. Das Fadenbündel 34a wird durch den Schlitz 29 in die Durchführungsöffnung 28 eingesteckt und auf der Wickelplatte 21 mäanderförmig verlegt. Dieses Verlegen erfolgt in der Wickelmaschine, die (nicht dargestellte) Dorne aufweist, welche zu beiden Enden der Wickelplatte 21 angeordnet sind. Um diese Dorne wird das Fadenbündel herumgelegt, so daß es den in Fig. 3 dargestellten Verlauf annimmt. Nach dem Verlegen des Fadenbündels wird die Überfaltplatte 23 über die Wickelplatte 21 gefaltet und die Lasche 25 wird in den Einsteckschlitz 21 eingesteckt, um das Fadenbündel zu sichern.

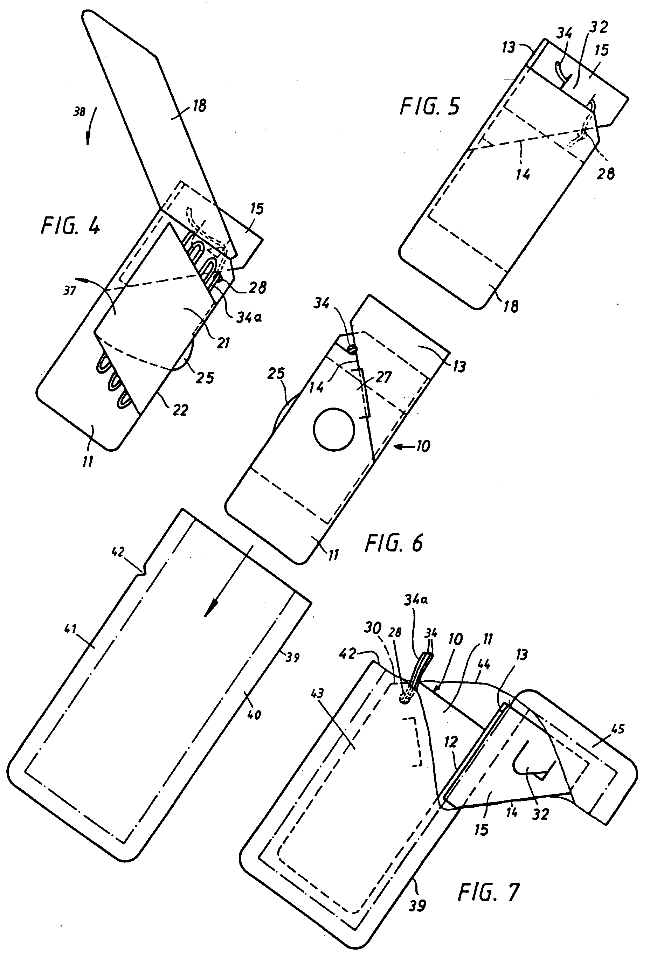

Fig. 4 zeigt das Umklappen der aufeinanderliegenden Wickelplatte 21 und Überfaltplatte 23 um die Faltlinie 22 herum auf die Basisplatte 11, wobei die Fadenhalteplatte 15 (zusammen mit der Faltplatte 13) das eine Ende der Basisplatte 11 überragt. Das Fadenbündel läuft ohne Knickung von der nach vorn weisenden Fixierlasche 32 durch die Durchführungsöffnung 28 hindurch in das Innere der von der Wickelplatte 21 und der Überfaltplatte 23 gebildeten Tasche, aus der die Fadenschlingen an beiden Enden herausragen. Das Umklappen der Fadentasche auf die Basisplatte 11 ist mit dem Pfeil 37 bezeichnet. Danach wird die Deckplatte 18 über die von der Wickelplatte und der Überfaltplatte gebildete Tasche gelegt (Pfeil 38).

Fig. 5 zeigt die zusammengefaltete Faltkarte von der Vorderseite, d.h. von derselben Seite, in der sie in den Fign. 1 bis 3 dargestellt ist. Die Faltkarte hat rechteckige, langgestreckte Form und sie ist etwas länger als die Basisplatte 11 bzw. die Deckplatte 18, weil die Fadenhalteplatte 15 mit der Faltplatte 13 die Basisplatte 11 und die Deckplatte 18 an einem Ende überragen. An diesem überstehenden Ende befindet sich die Fixierlasche 32, die die daruntergeklemmten Fadenenden festhält. Von dort verläuft das Fadenbündel durch die Durchführungsöffnung 28.

In Fig. 6 ist die gefaltete Faltkarte 10 von der Rückseite dargestellt, die von der Basisplatte 11 mit der daruntergefalteten Faltplatte 13 gebildet wird. Die von der schrägen Faltlinie 14 gebildete Faltkante ist unter die Verriegelungslasche 27 der Basisplatte 11 geschoben.

Die Faltkarte 10 wird gemäß Fig. 6 in die Hülle 39 eingeschoben, die aus einem Flachbeutel aus beschichteter Folie besteht, der längs seiner Kanten Siegelnähte 40 und 41 aufweist. Nach dem Einschieben der Faltkarte in die Beutelöffnung wird diese ebenfalls mit einer Siegelnaht verschlossen, so daß die Faltkarte mit dem Nähnahtmaterial vollständig umschlossen ist. Die Hülle 39 weist an ihrer einen Längskante eine Aufreißkerbe 42 auf, an der sie in Höhe der Abschrägungen 30,31 der Faltkarte aufgerissen werden kann. Beim Aufreißen gemäß Fig. 7 reißt die vordere Folie 43 der Hülle 39 längs der von der schrägen Faltlinie 14 gebildeten Faltkante auf, während die hintere Folie 44 unkontrolliert aufreißt. Dabei entsteht eine taschenförmige Aufreißtasche 45 der Folie 39. In dieser Tasche ist der über die Basisplatte 11 und die Deckplatte 18 überstehende Teil der Faltplatte 13 und der Fadenhalteplatte 15 enthalten. Die Faltplatte 13 wird um die Längskante 12 herum aufgeschwenkt. Dabei gleitet das Ende des Fadenbündelss 34a aus der Fixierlasche 32 heraus, um sich in der in Fig. 7 gezeigten Stellung anzubieten. Das Ende eines Fadens 34 braucht nur noch mit der Hand ergriffen zu werden, um den Faden aus der Faltkarte herauszuziehen. Dabei werden die anderen nicht ergriffenen Fäden 34 von der engen Durchführungsöffnung 28 zurückgehalten.

## Patentansprüche

1. Kombination von Nahtmaterialpackung und Nahtmaterial, mit einer das Nahtmaterial aufnehmenden, aus mehreren Platten bestehenden Faltkarte (10), in der das aus parallelen Einzelfäden (34) bestehende Nahtmaterial als mäanderförmiges Fadenbündel (34a) enthalten ist, dessen Fadenenden aus der Faltkarte herausragen,
**dadurch gekennzeichnet,**
daß eine Platte der Faltkarte (10) eine umfangsmäßig geschlossene Durchführungsöffnung (28) aufweist, von der sich ein Schlitz (29) zum benachbarten Rand dieser Platte erstreckt und durch die die Fadenenden des Fadenbündels (34a) verlaufen, wobei die Durchführungsöffnung (28) so eng bemessen ist, daß beim Herausziehen eines Einzelfadens (34) die anderen Fäden zurückgehalten werden, und daß das Fadenbündel (34a) in einer an entgegengesetzten Enden offenen Tasche festgelegt ist, die von einer Wickelplatte (21) und einer Überfaltplatte (23) gebildet und auf eine Basisplatte (11) umgefaltet ist, und daß die Durchführungsöffnung (28) in der Basisplatte (11) und/oder einer darüber faltbaren Deckplatte (18) vorgesehen ist.

2. Kombination nach Anspruch 1, dadurch gekennzeichnet, daß an der einen Seite der Durchführungsöffnung (28) eine Fadenhalteplatte (15) anliegt, welche eine Fixierlasche (32) für die Fadenenden aufweist.

3. Kombination nach Anspruch 2, dadurch gekennzeichnet, daß die Fadenhalteplatte (15) die Durchführungsöffnung (28) freiläßt.

## Claims

1. A combination of a suture material package and suture material, comprising a folding card (10) accommodating said suture material and consisting of a plurality of panels, the suture material, consisting of parallel individual threads (34), being contained in said folding card (10) in the manner of a serpentine bundle of threads (34a), with the thread ends thereof projecting out from the folding card,
**characterized in**
that a panel of said folding card (10) is provided with a cirumferentially closed passage opening (28) from which a slit (29) extends to the adjacent edge of said panel and which has the thread ends of said bundle of threads (34a) extending therethrough, said passage opening (28) being of a size sufficiently small to retain the other threads when one individual thread (34) is pulled out, that said bundle of threads (34a) is fixed in position within a pocket being open on opposite ends, which pocket is formed by a winding panel (21) and a fold-over panel (23) and is folded into a position over a base panel (11), and that said passage opening (28) is provided in said base panel (11) and/or in a cover panel (18) adapted to be folded onto the base panel (11).

2. The combination according to claim 1, characterized in that one side of said passage opening (28) has a thread holding panel (15) abutting thereon which is provided with a fixing flap (32) for the thread ends.

3. The combination according to claim 2, characterized in that said thread holding panel (15) leaves said passage opening (28) unobstructed.

## Revendications

1. Combinaison d'un emballage pour matériau de suture et d'un matériau de suture, comportant une carte pliable (10), qui reçoit le matériau de suture et est constituée de plusieurs panneaux et dans laquelle le matériau de suture constitué par des fils individuels parallèles (34) est logé sous la forme d'un écheveau sinueux de fils (34a), les extrémités des fils sortant hors de la carte pliable,
caractérisée en ce
qu'un panneau de la carte pliable (10) possède une ouverture de traversée (28) fermée sur sa circonférence, à partir de laquelle une fente (29) s'étend jusqu'au bord voisin de ce panneau et à travers laquelle passent les extrémités des fils de l'écheveau de fils (34a), l'ouverture de traversée (28) étant dimensionnée d'une manière suffisamment étroite pour que, lors de l'extraction d'un fil individuel (34), les autres fils soient retenus, et en ce que l'écheveau de fils (34a) est fixé dans une poche ouverte à des extrémités opposées, qui est formée par un panneau de bobinage (21) et un panneau de repliage superposé (23) et est repliée sur un panneau de base (11), et en ce que l'ouverture de traversée (28) est prévue dans le panneau de base (11) et/ou dans un panneau de couverture (18) pouvant être replié par-dessus le panneau de base.

2. Combinaison selon la revendication 1, caractérisée en ce que d'un côté de l'ouverture de traversée (28), est appliqué un panneau (15) de retenue des fils, qui possède une patte de fixation (32) pour les extrémités des fils.

3. Combinaison selon la revendication 2, caractérisée en ce que le panneau (15) de retenue des fils laisse l'ouverture de traversée (28) découverte.
